# EUROPÄISCHE PATENTANMELDUNG

(11) **EP 2 591 766 A2**
(43) Veröffentlichungstag der Anmeldung: **15.05.2013**
(21) Anmeldenummer: 12183179.6
(22) Anmeldetag: 05.09.2012
(51) Int. Cl.: A61K 8/41, A61Q 11/00

(54) **Mund- und Zahnpflege- und -reinigungsmittel mit Polyaminen**

(30) Priorität: 23.09.2011 DE 102011083324
(71) Anmelder: Henkel AG&Co. KGAA, 40589 Düsseldorf (DE)
(72) Erfinder: Miehlich, Kristin, 42119 Wuppertal (DE); Veith, Birgit, 40235 Düsseldorf (DE); Boy, Julia, 45479 Mülheim an der Ruhr (DE); Simmering, Rainer, 41515 Grevenbroich (DE); Breves, Roland, 40822 Mettmann (DE); Zelic, Daniela, 40883 Ratingen (DE)

(57) **Zusammenfassung**

Plaquereduzierende und Halitosis bekämpfende Mund- und Zahnpflege- und -reinigungsmittel enthalten - bezogen auf ihr Gewicht - 0,00001 bis 0,1 Gew.-% mindestens eines Polyamins der Formel (I) 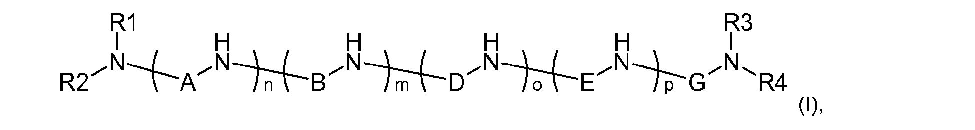 in der
R¹, R², R³ und R⁴
stehen unabhängig voneinander für ―H, -CH₃, -CH₂CH₃, -CH₂CH₂CH₃, -CH(CH₃)₂;
n, m, o, p
stehen unabhängig voneinander für 0, 1, 2, 3, 4, 5, wobei gilt: (n+m+o+p)>0;
A, B, D, E, G
stehen unabhängig voneinander für eine gesättigte oder ungesättigte Kohlenwasserstoffgruppierung.

## Beschreibung

Die Erfindung betrifft Mund- und Zahnpflegemittel zur Verringerung und Entfernung von Plaque und zur Vorbeugung vor Karies, die gleichzeitig die Zahnoberflächen gründlich reinigt und Mundgeruch sowie Parodontitis verringern hilft.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen und gleichzeitig Mundgeruch verringern und/oder verhindern.

In unzugänglichen Bereichen des Mundes wie beispielsweise in Zahnzwischenräumen oder die den hinteren Backenzähnen ist die herkömmliche mechanische Reinigung mit der Zahnbürste oft nicht vollständig erfolgreich, insbesondere dann, wenn die Putzdauer nicht ausreichend ist. Dies hat zur Folge, dass der Zahnbelag in diesen Bereichen nicht vollständig entfernt wird und sich Mikroorganismen wieder auf den Zähnen ansiedeln können, was zur Plaqueneubildung führt. Zahnbelag (Plaque) ist ein rauer, klebriger Belag auf den Zähnen, der aus Speichel, Bakterien und Nahrungsresten besteht. Setzen sich Mineralsalze (z.B. Calcium, Phosphat) aus dem Speichel im Zahnbelag ab, so bilden sich harte, weiße oder gelbliche Ablagerungen am Zahn, die man Zahnstein nennt. In dem porösen Zahnstein kann sich wiederum leicht Zahnbelag absetzen, der das Zahnfleisch angreift.

Die Bakterien auf der Zahnoberfläche bauen Kohlenhydrate, besonders Zucker, aus der Nahrung zu Säure ab. Diese Säure löst die Zahnsubstanz auf und es kommt zu Karies (Zahnfäule). Dabei werden besonders die Mineralien Calcium und Phosphat aus dem Zahnschmelz herausgelöst. Nach dem Zahnschmelzmantel werden auch innere Schichten des Zahnes angegriffen. Bakterien können in das Zahnmark eindringen und dort zu Entzündungen führen. Meist kommt es dann zu stechenden Zahnschmerzen.

Mundgeruch, auch Halitosis oder "Foetor ex ore" genannt, kann verschiedene Ursachen haben. Bei gesunden Menschen wird Mundgeruch jedoch in den meisten Fällen durch Bakterien im Mund- bzw. Rachenraum hervorgerufen, die Körperzellen oder Nahrungsreste verstoffwechseln, wobei als Abbauprodukte von Proteinen u.a. flüchtige Schwefelverbindungen ("volatile sulfur compounds", VSC) entstehen, die für den üblen Geruch verantwortlich gemacht werden.

Diese Schwefelverbindungen sind insbesondere: H₂S = Schwefelwasserstoff (zu etwa 30 %), CH₃-S-H = Methylmercaptan (zu etwa 60 %) und CH₃-S-CH₃ = Dimethylsulfid (zu etwa 10 %).

Die flüchtigen Schwefelverbindungen sind teilweise sehr aggressiv und können das Zahnfleisch und die Mundschleimhaut schädigen. So ist beispielsweise bekannt, dass Schwefelwasserstoff und Methylmercaptan die Durchlässigkeit der Mundschleimhaut erhöhen und so Zahnfleischerkrankungen Vorschub leisten können.

Es besteht nach wie vor das Bedürfnis, Plaque zu reduzieren, Halitosis zu bekämpfen und Wirkstoffe oder Wirkstoffkombinationen dafür bereitzustellen. Es bestand weiter die Aufgabe, Mund- und Zahnpflege- und -reinigungsmittel weiterzuentwickeln Mund- und Zahnpflege- und - reinigungsmittel bereitzustellen, die gegen Karies und Plaque wirksam sind. Dabei sollte nach Möglichkeit auf natürliche Inhaltsstoffe zurückgegriffen werden können, um einem gesteigerten Verbraucherbedürfnis nach "grünen" Produkten nachkommen zu können.

Es wurde nun gefunden, dass sich bestimmte Polyamine zur Lösung der genannten Aufgaben eignen.

Gegenstand der vorliegenden Erfindung ist in einer ersten Ausführungsform ein Mund- und Zahnpflege- und -reinigungsmittel, enthaltend - bezogen auf sein Gewicht - 0,00001 bis 0,1 Gew.-% mindestens eines Polyamins der Formel (I) 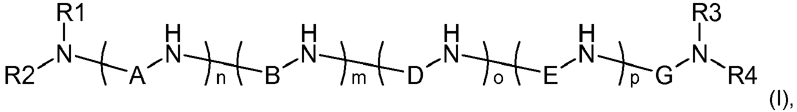 in der
- R¹, R², R³ und R⁴: stehen unabhängig voneinander für -H, -CH₃, -CH₂CH₃, -CH₂CH₂CH₃, -CH(CH₃)₂;
- n, m, o, p: stehen unabhängig voneinander für 0, 1, 2, 3, 4, 5, wobei gilt: (n + m + o + p) > 0;
- A, B, D, E, G: stehen unabhängig voneinander für eine gesättigte oder ungesättigte Kohlenwasserstoffgruppierung.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Als wesentlichen Bestandteil enthalten die erfindungsgemäßen Mund und Zahnpflege- und -reinigungsmittel mindestens ein Polyamin der Formel (I) in einer Menge von 0,00001 bis 0,1 Gew.-%, bezogen auf das Gesamtgewicht des erfindungsgemäßen Mund und Zahnpflege- und - reinigungsmittels.

Vorzugsweise enthalten die erfindungsgemäßen Mittel das bzw. die Polymain(e) der Formel (I) innerhalb engerer Mengenbeereiche, so dass bevorzugte erfindungsgemäße Mund- und Zahnpflege- und -reinigungsmittel dadurch gekennzeichnet sind, dass dies 0,0001 bis 0,09 Gew.-% vorzugsweise 0,001 bis 0,08 Gew.-% , weiter bevorzugt 0,01 bis 0,07 Gew.-%, noch weiter bevorzugt 0,02 bis 0,06 Gew.-% und insbesondere 0,025 bis 0,05 Gew.-% Polyamin(e) der Formel (I) enthalten.

Je nach Wahl der Reste R¹, R², R³ und R⁴, der Indices n, m, o und p und der Kohlenwasserstoffgruppierungen A, B, D, E und G können die erfindungsgemäß eingesetzten Polyamine Molmassen im Bereich von unter 100 Dalton bis hin zu mehreren hundert dalton aufweisen. Bevorzugte erfindungsgemäße Mittel enthalten dabei Polyamine mit Molmassen zwischen 100 und 1500 Dalton, vorzugsweise zwischen 110 und 1200 Dalton, besonders bevorzugt zwischen 120 und 1000 Dalton und insbesondere zwischen 125 und 500 Dalton.

Die Indices n, m, o, p stehen in Formel (I) unabhängig voneinander für 0, 1, 2, 3, 4, 5, wobei mindestens einer der Indices > 0 ist. In anderen Worten ist mindestens eine der Gruppierungen A, B, D oder E vorhanden.

Bevorzugt einzusetzende Polyamine der Formel (I) weisen folgende Indices auf:

| | | | | | | | |
|---|---|---|---|---|---|---|---|
| n= | 1 | m= | 0 | o= | 0 | p= | 0 |
| n= | 2 | m= | 0 | o= | 0 | p= | 0 |
| n= | 3 | m= | 0 | o= | 0 | p= | 0 |
| n= | 4 | m= | 0 | o= | 0 | p= | 0 |
| n= | 5 | m= | 0 | o= | 0 | p= | 0 |
| n= | 1 | m= | 1 | o= | 0 | p= | 0 |
| n= | 2 | m= | 1 | o= | 0 | p= | 0 |
| n= | 3 | m= | 1 | o= | 0 | p= | 0 |
| n= | 4 | m= | 1 | o= | 0 | p= | 0 |
| n= | 5 | m= | 1 | o= | 0 | p= | 0 |
| n= | 1 | m= | 0 | o= | 1 | p= | 0 |
| n= | 2 | m= | 0 | o= | 1 | p= | 0 |
| n= | 3 | m= | 0 | o= | 1 | p= | 0 |
| n= | 4 | m= | 0 | o= | 1 | p= | 0 |
| n= | 5 | m= | 0 | o= | 1 | p= | 0 |
| n= | 1 | m= | 1 | o= | 0 | p= | 1 |
| n= | 2 | m= | 1 | o= | 0 | p= | 1 |
| n= | 3 | m= | 1 | o= | 0 | p= | 1 |
| n= | 4 | m= | 1 | o= | 0 | p= | 1 |
| n= | 5 | m= | 1 | o= | 0 | p= | 1 |
| n= | 1 | m= | 1 | o= | 1 | p= | 1 |
| n= | 2 | m= | 1 | o= | 1 | p= | 1 |
| n= | 3 | m= | 1 | o= | 1 | p= | 1 |
| n= | 4 | m= | 1 | o= | 1 | p= | 1 |
| n= | 5 | m= | 1 | o= | 1 | p= | 1 |

Ganz besonders bevorzugt steht der Index n für die Zahl 1.

Weiter bevorzugt werden Polyamine eingesetzt, in denen m = p gilt.

Noch weiter bevorzugt werden Polyamine eingesetzt, in denen n für die Zahl 1 steht und m = p gilt. Noch weiter bevorzugt steht der Index o für die Zahl 0 oder 1, insbesondere für 0.

Ganz besonders bevorzugt werden Polyamine eingesetzt, in denen n für die Zahl 1 steht, o für die Zahl 0 oder 1 steht und m = p gilt.

Insbesondere bevorzugte erfindungsgemäße Mund- und Zahnpflege- und -reinigungsmittel enthalten 0,0001 bis 0,09 Gew.-% vorzugsweise 0,001 bis 0,08 Gew.-% , weiter bevorzugt 0,01 bis 0,07 Gew.-%, noch weiter bevorzugt 0,02 bis 0,06 Gew.-% und insbesondere 0,025 bis 0,05 Gew.-% Polyamin(e) der Formel (I), in der n für 1 steht und gleichzeitig m = p gilt.

Unabhängig von der Wahl der Indices n, m, o und p stehen die Reste R¹, R², R³ und R⁴ unabhängig voneinander für -H, -CH₃, -CH₂CH₃, -CH₂CH₂CH₃, -CH(CH₃)₂,

In einer bevorzugten Ausführungsform der vorliegenden Erfindung werden Verbindungen der Formel (I) eingesetzt, in der R¹ = R² und R³ = R⁴ gilt. Besonders bevorzugte erfindungsgemäße Mund- und Zahnpflege- und -reinigungsmittel enthalten daher mindestens eine Verbindung der Formel (I-a) und/oder (I-b) und/oder (I-c) und/oder (I-d) und/oder (I-e) und/oder (I-f) und/oder (I-g) und/oder (I-h) und/oder (I-i) und/oder (I-j) und/oder (I-k) und/oder (I-l) und/oder (I-m) und/oder (I-n) und/oder (I-o) und/oder (I-p) und/oder (I-q) und/oder (I-r) und/oder (I-s) und/oder (I-t) und/oder (I-u) und/oder (I-v) und/oder (I-w) und/oder (I-x) und/oder (I-y) wie im Prioritätsdokument auf den Seiten 5 bis 8 beschrieben.

In ganz besonders bevorzugten Verbindungen der Formel (I) gilt R¹ = R² = R³ = R⁴. Besonders bevorzugte erfindungsgemäße Mund- und Zahnpflege- und -reinigungsmittel enthalten daher mindestens eine Verbindung der Formel (I-a) und/oder (I-g) und/oder (I-m) und/oder (I-s) und/oder (I-y) wie im Prioritätsdokument auf den Seiten 5 bis 8 beschrieben.

In Formel (I) und den daraus abgeleiteten Formeln (I-a) bis (I-y) stehen n, m, o, p unabhängig voneinander für 0, 1, 2, 3, 4, 5, wobei (n + m + o + p) > 0 gilt. In anderen Worten ist mindestens einer der Indices n, m, o, p ≥ 1. Besonders bevorzugte Verbindungen sind dadurch gekennzeichnet, dass n für 1 steht.

In weiter bevorzugten Ausführungsformen der vorliegenden Erfindung gilt gleichzeitig m = p, so dass bevorzugte erfindungsgemäße Mittel Verbindungen der Formeln (I-a-0) bis (I-a-5) und/oder (I-g-0) bis (I-g-5) und/oder (I-m-0) bis (I-m-5) und/oder (I-s-0) bis (I-s-5) und/oder (I-y-0) bis (I-y-5) mit o = 0, 1, 2, 3, 4, oder 5 enthalten. Die entsprechenden Strukturformeln sind im Prioritätsdokument auf den Seiten 9 bis 12 beschrieben.

In ganz besonders bevorzugten Verbindungen der Formel (I) und den daraus abgeleiteten Formeln (I-a) bis (I-y) gilt n = 1 und m = p = 0, so dass besonders bevorzugte erfindungsgemäße Mund- und Zahnpflege- und -reinigungsmittel Verbindungen der Formeln (I-a-0) und/oder (I-g-0) und/oder (Im-0) und/oder (I-s-0) und/oder (I-y-0) mit o = 0, 1, 2, 3, 4, oder 5 enthalten.

Unter diesen wiederum insbesondere bevorzugt sind die Vertreter mit o = 0, so dass insbesondere bevorzugte erfindungsgemäße Mittel Verbindungen der Formeln (II-a) und/oder (II-g) und/oder (IIm) und/oder (II-s) und/oder (II-y) enthalten:

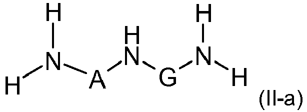

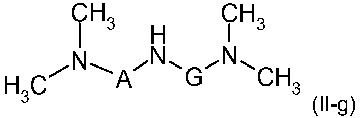

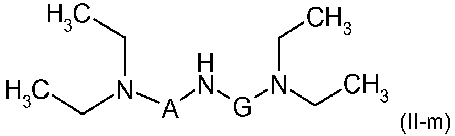

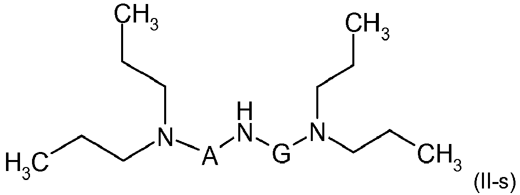

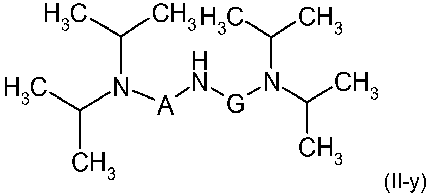

In Formel (I) und den daraus abgeleiteten Formeln (I-a) bis (I-y) bzw. (IIa) bis (II-y) stehen A, B, D, E und G unabhängig voneinander für eine gesättigte oder ungesättigte Kohlenwasserstoffgruppierung.

Unabhängig von der Wahl der Indices n, m, o und p und der Reste R¹, R², R³ und R⁴ stehen die verbrückenden Gruppierungen A, B, D, E und G unabhängig voneinander für eine gesättigte oder ungesättigte Kohlenwasserstoffgruppierung.

Vorzugsweise stehen A, B, D, E und G unabhängig voneinander für eine Gruppierung ausgewählt aus
- (CH₂)ₐ- mit a = 1, 2, 3, 4, 5, 6;
- (CH₂)_{b}-CH=CH-(CH₂)_{c}-, in der b und c unabhängig voneinander für 1, 2, 3, 4, 5, 6 stehen;
- (CH₂)_{d}-CH=CH-(CH₂)ₑ-CH=CH-(CH₂)_{f}-, in der d, e und f unabhängig voneinander für 1, 2, 3, 4, 5, 6 stehen;

Erfindungsgemäß bevorzugte Mund- und Zahnpflege- und -reinigungsmittel enthalten demnach 0,0001 bis 0,09 Gew.-% vorzugsweise 0,001 bis 0,08 Gew.-% , weiter bevorzugt 0,01 bis 0,07 Gew.-%, noch weiter bevorzugt 0,02 bis 0,06 Gew.-% und insbesondere 0,025 bis 0,05 Gew.-% Polyamin(e) der Formel (I), in der A, B, D, E, G unabhängig voneinander definiert sind wie folgt:
- (CH₂)ₐ- mit a = 1, 2, 3, 4, 5, 6;
- (CH₂)_{b}-CH=CH-(CH₂)_{c}-, in der b und c unabhängig voneinander für 1, 2, 3, 4, 5, 6 stehen;
- (CH₂)_{d}-CH=CH-(CH₂)ₑ-CH=CH-(CH₂)_{f}-, in der d, e und f unabhängig voneinander für 1, 2, 3, 4, 5, 6 stehen.

Ganz besonders bevorzugte Gruppierungen A, B, D, E und G sind -(CH₂)₃-, -(CH₂)₄-, -(CH₂)-CH=CH-(CH₂)-, und -(CH₂)-CH=CH-(CH₂)-CH=CH-(CH₂)-, insbesondere bevorzugt sind -(CH₂)₃- und -(CH₂)₄-, so dass bevorzugte erfindungsgemäße Mittel Verbindungen der Formeln (II-a-1) bis (II-a-3) und/oder (II-g-1) bis (II-g-3) und/oder (II-m-1) bis (II-mn-3) und/oder (II-s-1) bis (II-s-3) und/oder (II-y-1) bis (II-y-3) enthalten:

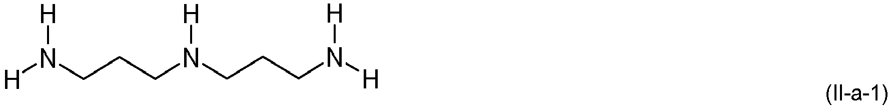

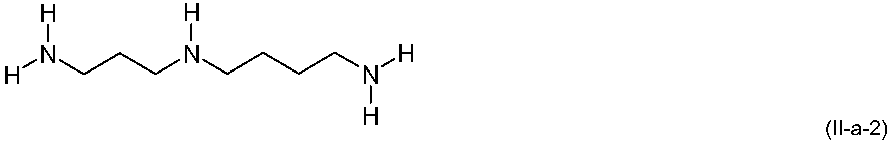

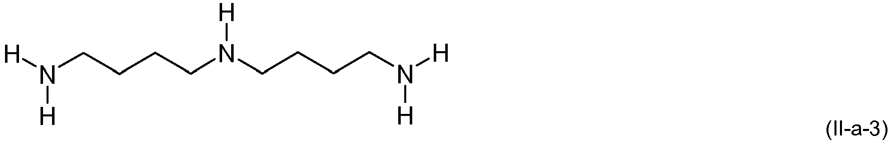

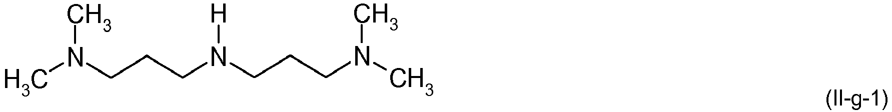

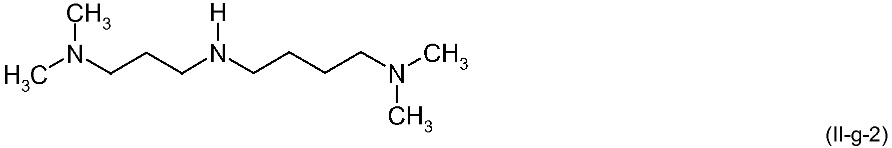

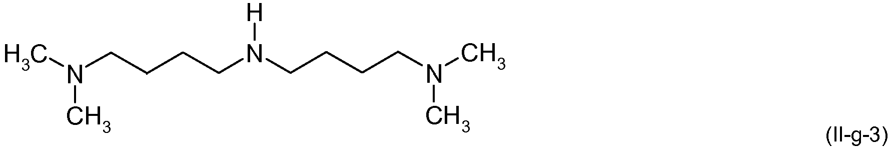

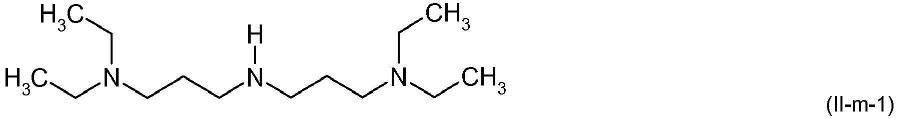

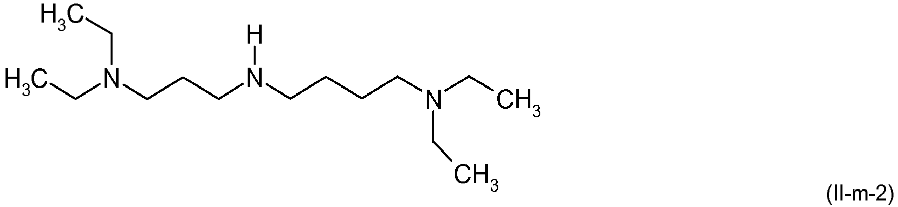

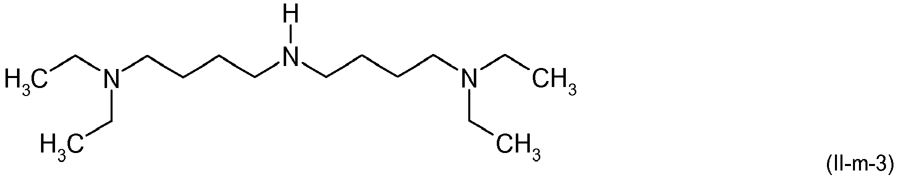

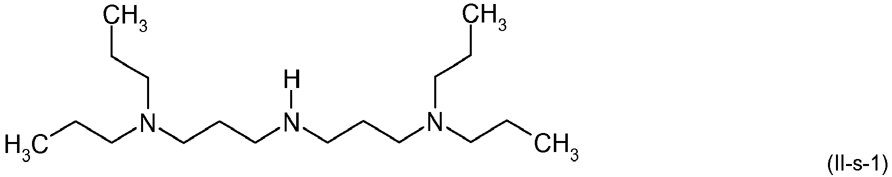

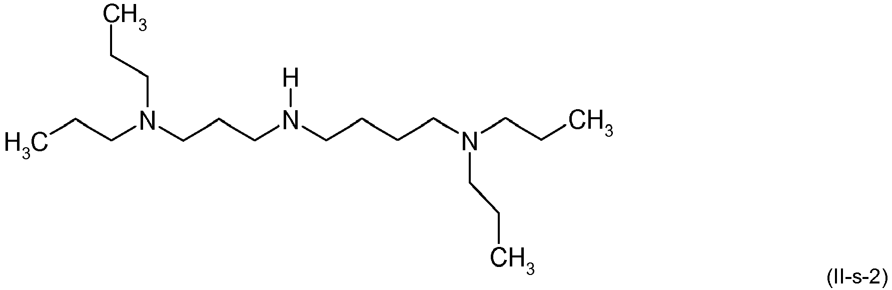

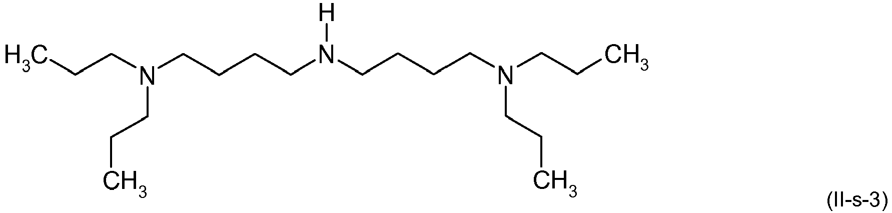

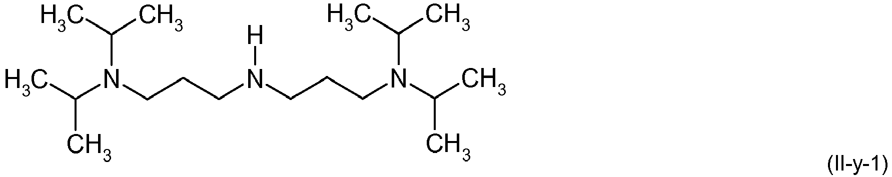

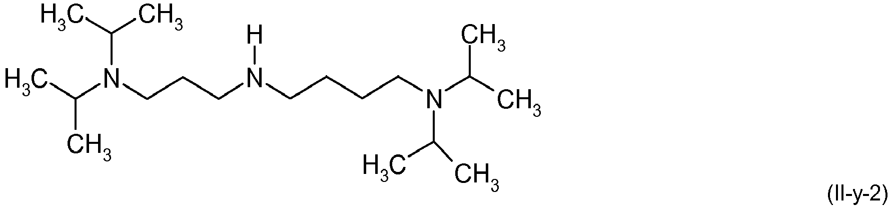

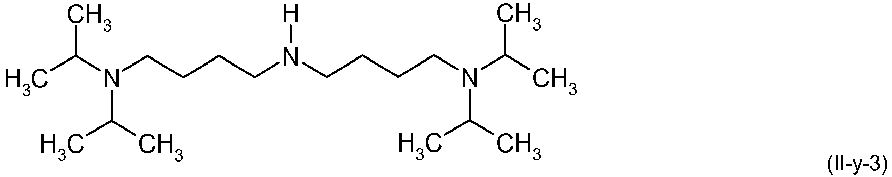

Besonders bevorzugte erfindungsgemäße Mund- und Zahnpflege- und -reinigungsmittel enthalten 0,0001 bis 0,09 Gew.-% vorzugsweise 0,001 bis 0,08 Gew.-% , weiter bevorzugt 0,01 bis 0,07 Gew.-%, noch weiter bevorzugt 0,02 bis 0,06 Gew.-% und insbesondere 0,025 bis 0,05 Gew.-% Polyamin(e) der Formel (I), in der gilt:
- R¹ = R² = R³ = R⁴ = -H
- n = 1
- m = o = p = 0
- A und G sind unabhängig voneinander ausgewählt aus -(CH₂)₃-, -(CH₂)₄-, -(CH₂)-CH=CH-(CH₂)-, und -(CH₂)-CH=CH-(CH₂)-CH=CH-(CH₂)-.

Ebenfalls bevorzugt sind erfindungsgemäße Mund- und Zahnpflege- und -reinigungsmittel, die Verbindungen der Formeln (I-a-0) und/oder (I-g-0) und/oder (I-m-0) und/oder (I-s-0) und/oder (I-y-0) mit o = 1 enthalten. Diese insbesondere bevorzugten erfindungsgemäßen Mittel enthalten Verbindungen der Formeln (III-a) und/oder (III-g) und/oder (III-m) und/oder (III-s) und/oder (III-y):

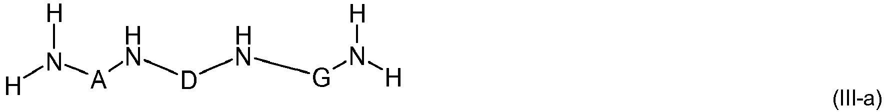

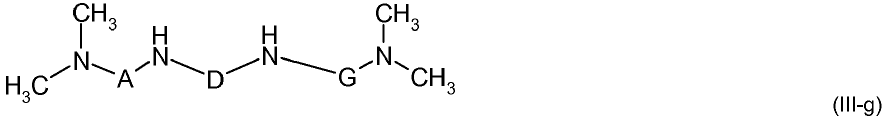

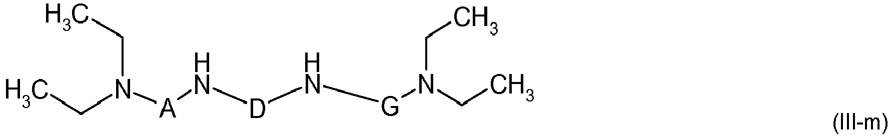

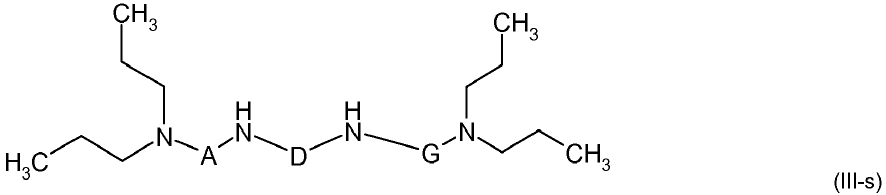

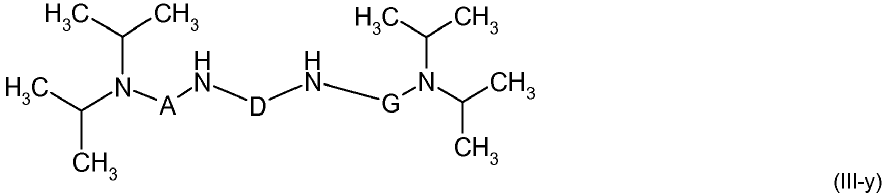

Auch in den aus Formel (I) abgeleiteten Formeln (III-a) bis (III-y) stehen A, B, D, E und G unabhängig voneinander für eine gesättigte oder ungesättigte Kohlenwasserstoffgruppierung. Vorzugsweise stehen A, B, D, E und G auch hier unabhängig voneinander für eine Gruppierung ausgewählt aus
- (CH₂)ₐ- mit a = 1, 2, 3, 4, 5, 6;
- (CH₂)_{b}-CH=CH-(CH₂)_{c}-, in der b und c unabhängig voneinander für 1, 2, 3, 4, 5, 6 stehen;
- (CH₂)_{d}-CH=CH-(CH₂)ₑ-CH=CH-(CH₂)_{f}-, in der d, e und f unabhängig voneinander für 1, 2, 3, 4, 5, 6 stehen;

Ganz besonders bevorzugte Gruppierungen A, B, D, E und G sind -(CH₂)₃-, -(CH₂)₄-, -(CH₂)-CH=CH-(CH₂)-, und -(CH₂)-CH=CH-(CH₂)-CH=CH-(CH₂)-, insbesondere bevorzugt sind -(CH₂)₃- und -(CH₂)₄-, so dass bevorzugte erfindungsgemäße Mittel Verbindungen der Formeln (III-a-1) bis (III-a-6) und/oder (III-g-1) bis (III-g-6) und/oder (III-m-1) bis (III-mn-6) und/oder (III-s-1) bis (III-s-6) und/oder (III-y-1) bis (III-y-6) enthalten:

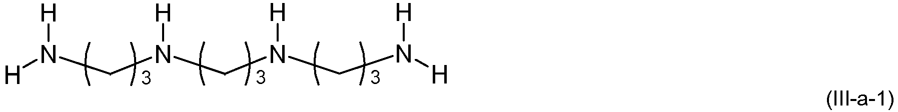

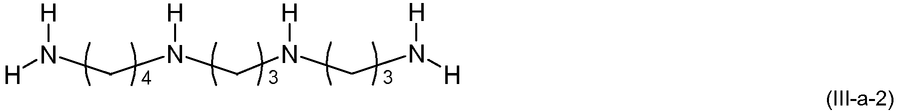

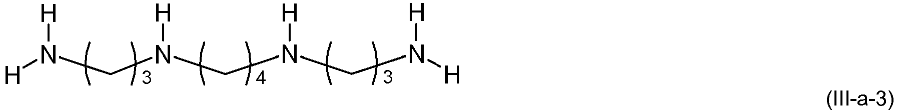

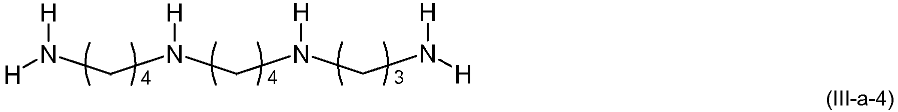

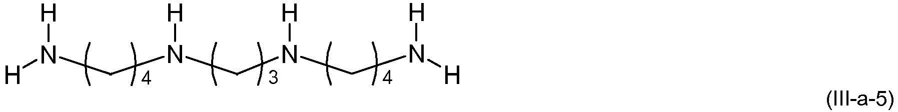

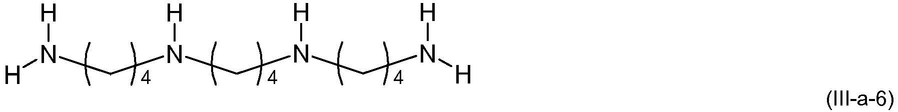

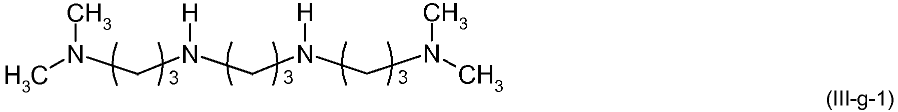

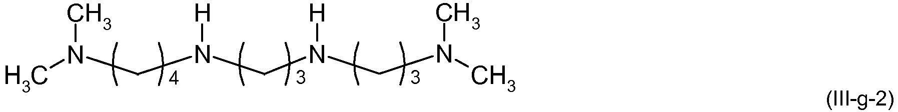

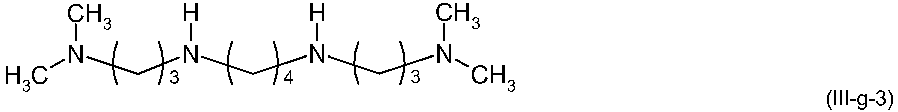

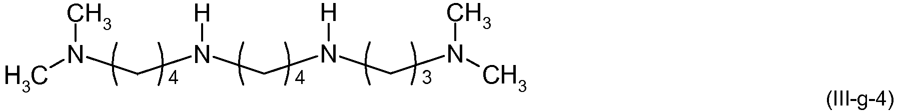

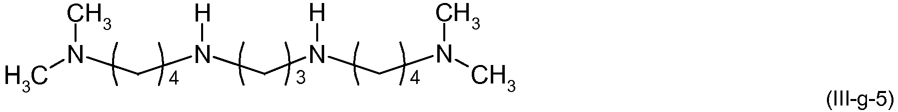

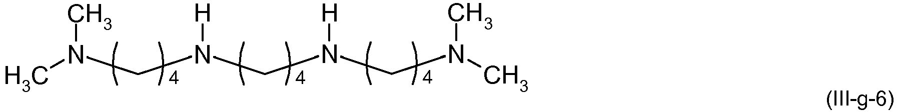

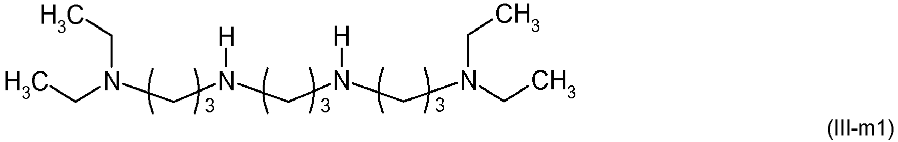

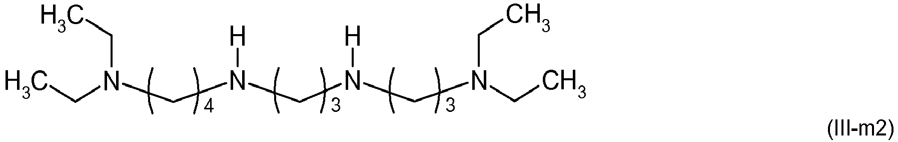

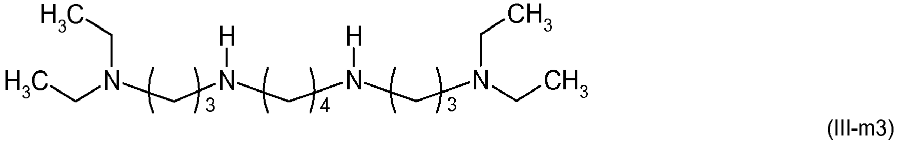

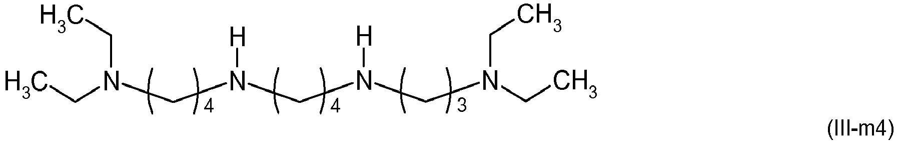

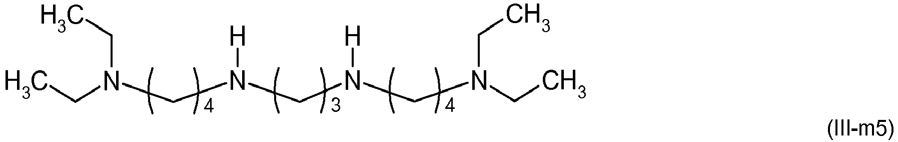

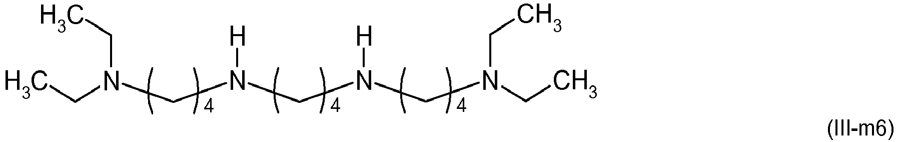

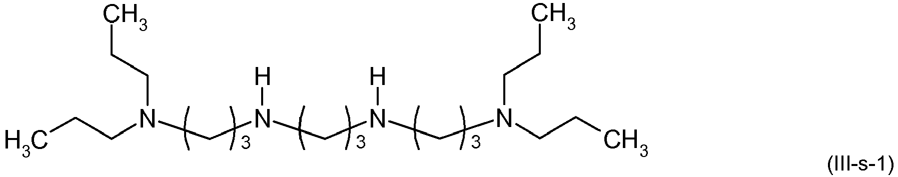

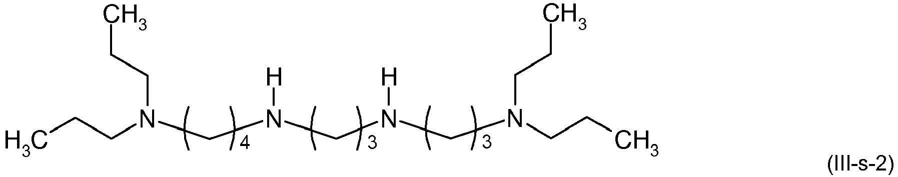

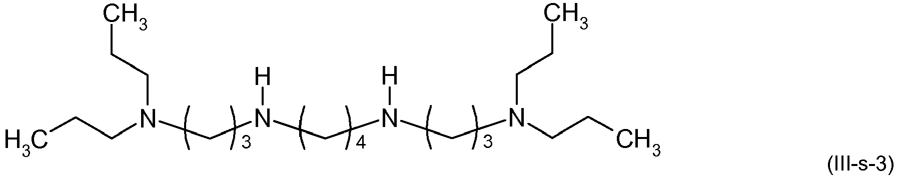

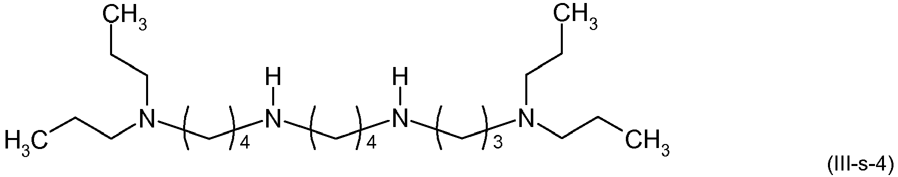

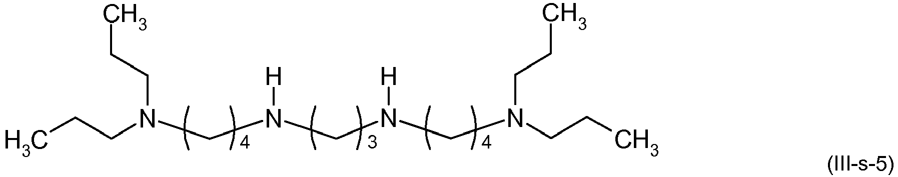

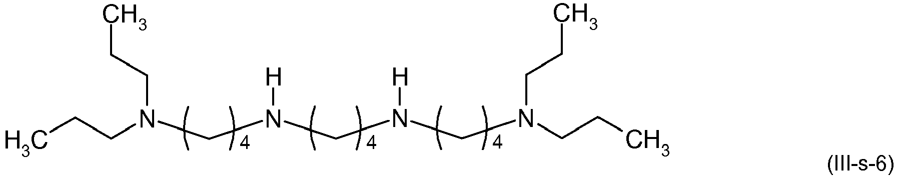

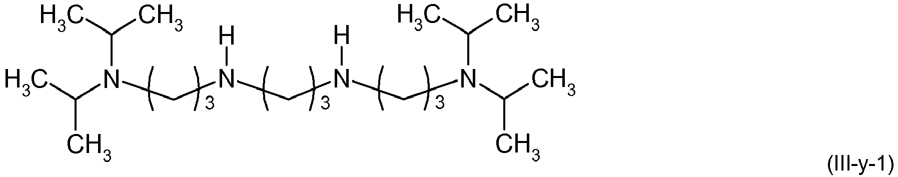

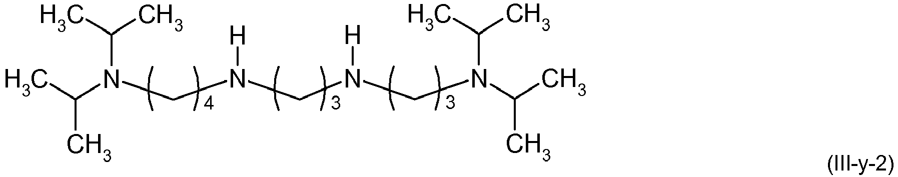

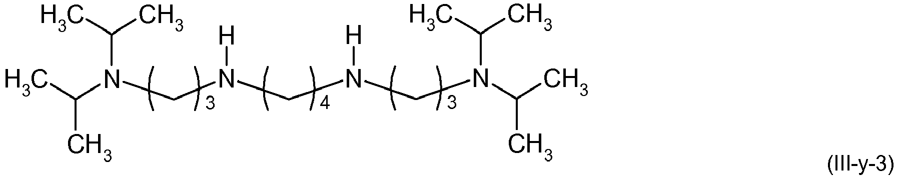

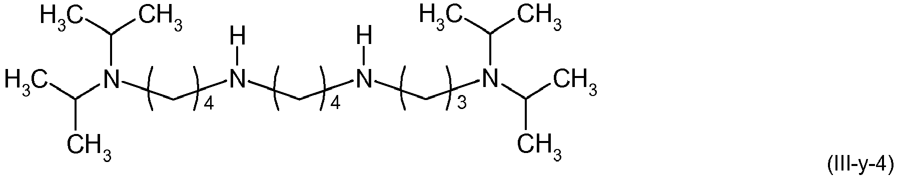

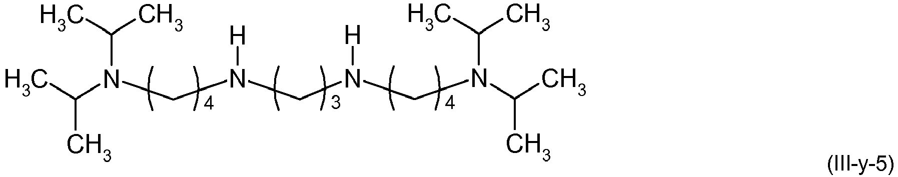

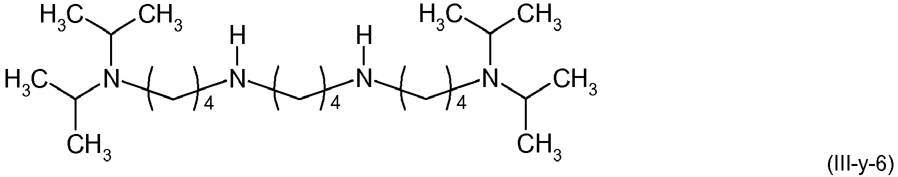

Zusammenfassend sind besonders bevorzugte erfindungsgemäße Mund- und Zahnpflege- und - reinigungsmittel dadurch gekennzeichnet, dass sie 0,0001 bis 0,09 Gew.-% vorzugsweise 0,001 bis 0,08 Gew.-% , weiter bevorzugt 0,01 bis 0,07 Gew.-%, noch weiter bevorzugt 0,02 bis 0,06 Gew.-% und insbesondere 0,025 bis 0,05 Gew.-% Polymain(e) der Formeln 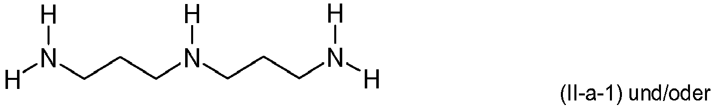 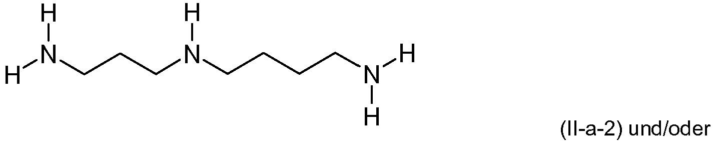 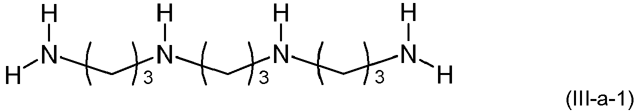 und/oder deren Mischungen enthalten.

In einer weiteren bevorzugten Ausführungsform der vorliegenden Erfindung ist R¹ = R³ und R² = R⁴. Besonders bevorzugte Varianten dieser Ausführungsform sind dadurch gekennzeichnet, dass R¹ = R³ = -H und R² = R⁴ = -CH₂CH₃ gilt. Besonders bevorzugte erfindungsgemäße Mund- und Zahnpflege- und -reinigungsmittel enthalten daher Verbindungen der Formel (IV) 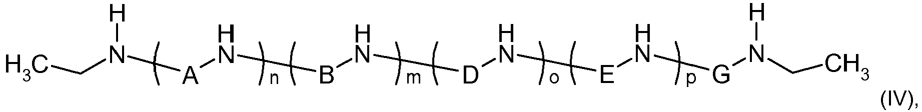 in der A, B, D, E und G sowie n, m, o und p definiert sind, wie vorstehend beschrieben. In dieser bevorzugten Ausführungsform ist es wiederum bevorzugt, wenn n = m = o = 1 und p = 0 sind, weshalb besonders bevorzugte erfindungsgemäße Mund- und Zahnpflege- und -reinigungsmittel Verbindungen der Formel (IVa) enthalten 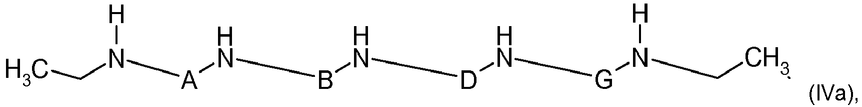 in der A, B, D und G definiert sind, wie vorstehend beschrieben. Auch Formeln (IV) und (IVa) stehen A, B, D, E und G unabhängig voneinander für eine gesättigte oder ungesättigte Kohlenwasserstoffgruppierung. Vorzugsweise stehen A, B, D, E und G auch hier unabhängig voneinander für eine Gruppierung ausgewählt aus
- (CH₂)ₐ- mit a = 1, 2, 3, 4, 5, 6;
- (CH₂)_{b}-CH=CH-(CH₂)_{c}-, in der b und c unabhängig voneinander für 1, 2, 3, 4, 5, 6 stehen;
- (CH₂)_{d}-CH=CH-(CH₂)ₑ-CH=CH-(CH₂)_{f}-, in der d, e und f unabhängig voneinander für 1, 2, 3, 4, 5, 6 stehen;

Ganz besonders bevorzugte Gruppierungen A, B, D, E und G sind -(CH₂)₃-, -(CH₂)₄-, -(CH₂)-CH=CH-(CH₂)-, und -(CH₂)-CH=CH-(CH₂)-CH=CH-(CH₂)-, insbesondere bevorzugt sind -(CH₂)₄- und -(CH₂)-CH=CH-(CH₂)-, so dass bevorzugte erfindungsgemäße Mittel Verbindungen der Formel (IVa) enthalten, in der gilt:

A = B = D = G = -(CH₂)₄- (IVa-1)

A = B = D = -(CH₂)₄-, G = -(CH₂)-CH=CH-(CH₂)- (IVa-2)

A = B = G = -(CH₂)₄-, D = -(CH₂)-CH=CH-(CH₂)- (IVa-3)

A = B = -(CH₂)₄-, D = G = -(CH₂)-CH=CH-(CH₂)- (IVa-4)

A = G = -(CH₂)₄-, D = B = -(CH₂)-CH=CH-(CH₂)- (IVa-5)

D = B = -(CH₂)₄-, A = G = -(CH₂)-CH=CH-(CH₂)- (IVa-6)

A = D = -(CH₂)₄-, B = G = -(CH₂)-CH=CH-(CH₂)- (IVa-7)

A = -(CH₂)₄-, B = D = G = -(CH₂)-CH=CH-(CH₂)- (IVa-8)

B = -(CH₂)₄-, A = D = G = -(CH₂)-CH=CH-(CH₂)- (IVa-9)

A = B = D = G = -(CH₂)-CH=CH-(CH₂)- (IVa-10)

Zusammenfassend sind ebenfalls besonders bevorzugte erfindungsgemäße Mund- und Zahnpflege- und -reinigungsmittel dadurch gekennzeichnet, dass sie 0,0001 bis 0,09 Gew.-% vorzugsweise 0,001 bis 0,08 Gew.-% , weiter bevorzugt 0,01 bis 0,07 Gew.-%, noch weiter bevorzugt 0,02 bis 0,06 Gew.-% und insbesondere 0,025 bis 0,05 Gew.-% Polymain(e) der Formel (IVa-1) bis (IVa-10) und/oder deren Mischungen enthalten: 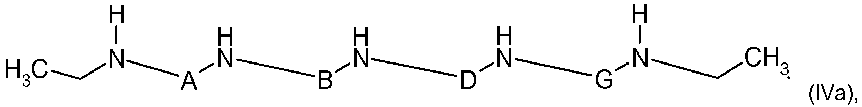 in der gilt:

A = B = D = G = -(CH₂)₄- (IVa-1) und/oder

A = B = D = -(CH₂)₄-, G = -(CH₂)-CH=CH-(CH₂)- (IVa-2) und/oder

A = B = G = -(CH₂)₄-, D = -(CH₂)-CH=CH-(CH₂)- (IVa-3) und/oder

A = B = -(CH₂)₄-, D = G = -(CH₂)-CH=CH-(CH₂)- (IVa-4) und/oder

A = G = -(CH₂)₄-, D = B = -(CH₂)-CH=CH-(CH₂)- (IVa-5) und/oder

D = B = -(CH₂)₄-, A = G = -(CH₂)-CH=CH-(CH₂)- (IVa-6) und/oder

A = D = -(CH₂)₄-, B = G = -(CH₂)-CH=CH-(CH₂)- (IVa-7) und/oder

A = -(CH₂)₄-, B = D = G = -(CH₂)-CH=CH-(CH₂)- (IVa-8) und/oder

B = -(CH₂)₄-, A = D = G = -(CH₂)-CH=CH-(CH₂)- (IVa-9) und/oder

A = B = D = G = -(CH₂)-CH=CH-(CH₂)- (IVa-10).

Die erfindungsgemäßen Mittel enthalten das/die Polyamin(e) und je nach Konfektionierung der erfindungsgemäßen Mittel weitere übliche Inhaltsstoffe von Mund- und Zahnpflege- und -reinigungsmitteln.

Eine wichtige Gruppe von Inhaltsstoffen stellen dabei die Tenside dar. Mit besonderem Vorzug können die erfindungsgemäßen Mittel anionische(s) Tensid(e) enthalten. Erfindungsgemäß bevorzugte Mund und Zahnpflege- und -reinigungsmittel sind dadurch gekennzeichnet, dass sie - bezogen auf ihr Gewicht - 0,25 bis 4 Gew.-%, vorzugsweise 0,5 bis 3,5 Gew.-%, weiter bevorzugt 0,75 bis 3 Gew.-%, noch weiter bevorzugt 1 bis 2,5 Gew.-% und insbesondere 1,6 bis 2,2 Gew.-% anionische(s) Tensid(e) enthalten.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Ganz besonders bevorzugte erfindungsgemäße Mittel enthalten Alkylsulfat(e) als anionisches Tensid. Hier sind erfindungsgemäß besonders bevorzugte Mund und Zahnpflege- und -reinigungsmittel dadurch gekennzeichnet, dass sie 0,25 bis 4 Gew.-%, vorzugsweise 0,5 bis 3,5 Gew.-%, weiter bevorzugt 0,75 bis 3 Gew.-%, noch weiter bevorzugt 1 bis 2,5 Gew.-% und insbesondere 1,6 bis 2,2 Gew.-% Natriumdodecylsulfat enthalten.

Als weiteren Inhaltsstoff können die erfindungsgemäßen Mittel amphotere(s) Tensid(e) enthalten. Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Grupp enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Besonders bevorzugte erfindungsgemäße Mund und Zahnpflege- und -reinigungsmittel sind dadurch gekennzeichnet, dass sie - bezogen auf ihr Gewicht - 0,01 bis 2 Gew.-%, vorzugsweise 0,05 bis 1,5 Gew.-%, weiter bevorzugt 0,1 bis 1 Gew.-%, noch weiter bevorzugt 0,12 bis 0,7 Gew.-% und insbesondere 0,15 bis 0,6 Gew.-% amphotere(s) Tensid(e) enthalten.

Besonders bevorzugte erfindungsgemäße Mund und Zahnpflege- und -reinigungsmittel sind dadurch gekennzeichnet, dass sie amphotere(s) Tensid(e) aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂ - C₁₈ - Acylsarcosin,
- N-Alkyl-N,N-dimethylammonium-glycinate, beispielsweise Kokosalkyl-dimethylammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Cocamidopropyl Betain bekannten Verbindungen,
- der unter der INCI-Bezeichnung Disodium Cocoamphodiacetate bekannten Verbindungen
enthalten.

Besonders bevorzugte Mund und Zahnpflege- und -reinigungsmittel enthalten als amphotere Tenside Betaine der Formel (Bet-I) 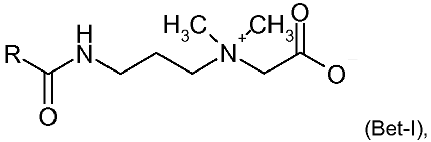 in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (Bet-I) eingesetzt, die ein Gemisch der folgenden Vertreter sind:

H₃C-(CH₂)₇-C(O)-NH-(CH₂)₃N⁺(CH₃)₂CH₂COO⁻

H₃C-(CH₂)₉-C(O)-NH-(CH₂)₃N⁺(CH₃)₂CH₂COO⁻

H₃C-(CH₂)₁₁-C(O)-NH-(CH₂)₃N⁺(CH₃)₂CH₂COO⁻

H₃C-(CH₂)₁₃-C(O)-NH-(CH₂)₃N⁺(CH₃)₂CH₂COO⁻

H₃C-(CH₂)₁₅-C(O)-NH-(CH₂)₃N⁺(CH₃)₂CH₂COO⁻

H₃C-(CH₂)₇-CH=CH-(CH₂)₇-C(O)-NH-(CH₂)₃N⁺(CH₃)₂CH₂COO⁻

Besonders bevorzugt werden Tenside der Formel (Bet-I) innerhalb engerer Mengenbereiche eingesetzt. Hier sind erfindungsgemäße Mund und Zahnpflege- und -reinigungsmittel bevorzugt, die - bezogen auf ihr Gewicht - 0,01 bis 2 Gew.-%, vorzugsweise 0,05 bis 1,5 Gew.-%, weiter bevorzugt 0,1 bis 1 Gew.-%, noch weiter bevorzugt 0,12 bis 0,7 Gew.-% und insbesondere 0,15 bis 0,6 Gew.-% Cocoamidopropylbetaine enthalten.

In besonders bevorzugten erfindungsgemäßen Mitteln ist die Gesamtmenge an Tensiden ebenfalls begrenzt. Hier sind erfindungsgemäße Mund und Zahnpflege- und -reinigungsmittel bevorzugt, bei denen die Gesamtmenge an Tensiden 0,5 bis 5 Gew.-%, vorzugsweise 0,75 bis 4,5 Gew.-%, weiter bevorzugt 1 bis 4 Gew.-%, noch weiter bevorzugt 1,25 bis 3,5 Gew.-% und insbesondere 1,6 bis 2,5 Gew.-% beträgt.

Die erfindungsgemäßen Zusammensetzungen enthalten vorzugsweise mindestens ein Poliermittel. Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels. Erfindungsgemäß bevorzugte Mittel enthalten Poliermittel innerhalb engerer Mengenbereiche. Hier sind erfindungsgemäß bevorzugte Mund- und Zahnpflege- und - reinigungsmittel dadurch gekennzeichnet, dass sie - bezogen auf ihr Gewicht - 1 bis 25 Gew.-%, vorzugsweise 2,5 bis 20 Gew.-%, weiter bevorzugt 5 bis 18 Gew.-% und insbesondere 7,5 bis 16 Gew.-% Poliermittel enthalten.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wässrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die so genannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident^{®}12 DS (DEGUSSA) erhältlich.

Andere Fällungskieselsäuren dieser Art sind Sident^{®} 8 (DEGUSSA) und Sorbosil^{®} AC 39 (Crosfield Chemicals). Diese Kieselsäuren zeichnen sich durch eine geringere Verdickungswirkung und eine etwas höhere mittlere Teilchengröße von 8 --14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungs-Kieselsäuren mit einer BET-Oberfläche von 150 --250 m²/g zu, z.B. die Handelsprodukte Sipernat 22 LS oder Sipernat^{®} 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Erfindungsgemäß besonders bevorzugte Mund und Zahnpflege- und -reinigungsmittel enthalten ausschließlich Poliermittel aus der Gruppe der Kieselsäuren, Aluminiumhydroxid, Aluminiumoxid, Dicalciumphosphat-dihydrat (CaHPO₄ ·2H₂O) oder Gemische dieser Reibkörper. Diese Poliermittel haben ich als besonders effizient bei der Lösung der erfindungsgemäßen Aufgabe erwiesen. Ganz besonders bevorzugt enthalten die erfindungsgemäßen Zusammensetzungen bezogen auf ihr Gewicht 1 bis 30 Gew.-% Fällungskieselsäure(n) mit einer spezifischen Oberfläche nach ISO 5794-1, Anhang D von ≤ 55 m²/g. Vorzugsweise werden die Fällungskieselsäuren, die entsprechende spezifische Oberflächen aufweisen, innerhalb engerer Mengenbereiche eingesetzt, und insbesondere bevorzugt werden Fällungskieselsäuren eingesetzt, die noch niedrigere spezifische Oberflächen nach ISO 5794-1, Anhang D aufweisen. Bevorzugte erfindungsgemäße Mund- und Zahnpflege- und -reinigungsmittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 13,0 Gew.-% Fällungskieselsäure(n) mit einer spezifischen Oberfläche nach ISO 5794-1, Anhang D von ≤ 55 m²/g.

Besonders bevorzugte erfinddungsgemäße Mund und Zahnpflege- und -reinigungsmittel sind dadurch gekennzeichnet, dass sämtliche im Mittel enthaltenen Fällungskieselsäure(n) eine spezifische Oberfläche nach ISO 5794-1, Anhang D von ≤ 53 m²/g, vorzugsweise von ≤ 51 m²/g, weiter bevorzugt von ≤ 49 m²/g und insbesondere von ≤ 47 m²/g aufweisen.

In weiter bevorzugten erfindungsgemäßen Mitteln sind die eingesetzten Fällungskieselsäuren durch weitere physikalische Parameter gekennzeichnet. Vorzugsweise einzusetzende Fällungskieselsäuren weisen Stampfdichten > 360 g/l (gemessen nach ISO 787-11), besonders bevorzugt > 375 g/l, weiter bevorzugt > 400 g/l und insbesondere > 425 g/l auf.

Es ist weiter bevorzugt, Fällungskieselsäuren einzusetzen, die eine DBP-Absorption gemäß DIN 53601 von weniger als 140 g/100 g aufweisen. Ganz besonders bevorzugte erfindungsgemäß einzusetzende Fällungskieselsäuren weisen eine DBP-Absorption gemäß DIN 53601 von weniger als 135 g/100 g, bevorzugt von eine DBP-Absorption gemäß DIN 53601 von weniger als 130 g/100 g und insbesondere von weniger als 125 g/100 g auf.

Erfindungsgemäß besonders bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l.

Erfindungsgemäß weiter bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer DBP-Absorption gemäß DIN 53601 von weniger als 125 g/100 g.

Erfindungsgemäß insbesondere bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l und einer DBP-Absorption gemäß DIN 53601 von weniger als 125 g/100 g.

Zusätzlich zu den erfindungsgemäß eingesetzten Polyaminen können Mund- und Zahnpflege- und reinigungsmittel, insbesondere Zahnpasten, auch weitere Substanzen enthalten, die gegen Plaque und/oder Zahnstein wirksam sind.

Wie bereits erwähnt, beinhaltet Plaque Bakterien, so dass sich zur Bekämpfung von Plaque antimikrobielle Stoffe eignen. Diese besitzen darüber hinaus eine Wirkung als Konservierungsmittel. In Mund und Zahnpflege- und -reinigungsmitteln können beispielsweise die auch in Lebensmitteln zugelassenen Konservierungsmittel Sorbinsäure (E 200), Kaliumsorbat (E 202), Calciumsorbat (E 203), Benzoesäure (E 210), Natriumbenzoat (E 211), Kaliumbenzoat (E 212), Calciumbenzoat (E 213), Ethyl-4-hydroxybenzoat (E 214), Ethyl-4-hydroxybenzoat, Natriumsalz (E 215), Propyl-4-hydroxybenzoat (E 216), Propyl-4-hydroxybenzoat, Natriumsalz (E 217), Methyl-4-hydroxybenzoat (E 218), Methyl-4-hydroxybenzoat, Natriumsalz (E 219), Schwefeldioxid (schweflige Säure), (E 220), Natriumsulfit (E 221), Natriumhydrogensulfit (E 222), Natriumdisulfit (E 223), Kaliumdisulfit (E 224), Calciumsulfit (E 226), Calciumhydrogensulfit (E 227), Kaliumhydrogensulfit (E 228), Biphenyl (E 230), Orthophenylphenol (2-Biphenylol), (E 231), Natriumorthophenylphenolat (E 232), Nisin (E 234), Natamycin (E 235), Ameisensäure (E 236), Natriumformiat (E 237), Calciumformiat (E 238), Hexamethylentetramin (E 239), Dimethyldicarbonat (E 242), Kaliumnitrit (E 249), Natriumnitrit (E 250), Natriumnitrat (E 251), Essigsäure (E 260), Kaliumacetat (E 261), Natriumacetat (E 262), Calciumacetat (E 263), Milchsäure (E 270), Propionsäure (E 280), Natriumpropionat (E 281), Calciumpropionat (E 282), Kaliumpropionat (E 283), Borsäure (E 284), Natriumtetraborat (E 285), Hydroxybernsteinsäure (Äpfelsäure), (E 296), Fumarsäure (E 297), Lysozym (E 1105), eingesetzt werden.

Bevorzugte Stoffe sind ausgewählt aus Alkypyridiniumsalzen, insbesondere Cetylpyridiniumchlorid, p-Hydroxybenzoesäuremethyl-, -ethyl- oder propylester, Natriumsorbat, Natriumbenzoat, Bromchlorophen, Triclosan, Phenyl- Salicylsäureester, Biguaniden z. B. Chlorhexidin (1,1'-Hexamethylenbis[5-(4-chlorphenyl)-biguanid), Thymol usw..

Erfindungsgemäß bevorzugte Mund- und Zahnpflege- und -reinigungsmittel sind dadurch gekennzeichnet, dass sie zusätzlich Antiplaque-Wirkstoffe, vorzugsweise Alkypyridiniumsalze, insbesondere Cetylpyridiniumchlorid, p-Hydroxybenzoesäuremethyl-, -ethyl- oder propylester, Natriumsorbat, Natriumbenzoat, Bromchlorophen, Triclosan, Hexetidine, Phenyl- Salicylsäureester, Biguanide z. B. Chlorhexidin, Thymol, vorzugsweise in Mengen von 0,05 bis 5 Gew.%, vorzugsweise von 0,10 bis 2,5 Gew.% und insbesondere von 0,30 bis 1,5 Gew.%, jeweils bezogen auf das gesamte Mittel, enthalten.

Die angegebenen Mengen beziehen sich auf die Gesamtmenge an den vorstehend genannten Antiplaque-Wirkstoffen, wobei einerseits die erfindungsgemäß eingesetzten Polyamine nicht eingerechnet werden und andererseits einzelne Wirkstoffe vorzugsweise in engeren Mengenbereichen eingesetzt werden. Erfindungsgemäß bevorzugte Mittel enthalten beispielsweise 0,1 bis 2 Gew.-%, vorzugsweise 0,2 bis 1,75 Gew.-%, weiter bevorzugt 0,3 bis 1,5 Gew.-% und insbesondere 0,4 bis 1,0 Gew.-% Natriumbenzoat. Weiter bevorzugte erfindungsgemäße Mittel sind dadurch gekennzeichnet, dass sie 0,005 bis 0,1 Gew.-%, vorzugsweise 0,01 bis 0,075 Gew.-% und insbesondere 0,02 bis 0,05 Gew.-% Chlorhexidin enthalten. Chlorhexidin wird vorzugsweise gemeinsam mit Alkylpolyglycosiden (APG) eingesetzt, wobei in bevorzugten erfindungsgemäßen Mitteln als Alkylglykoside solche mit 8-18 C-Atomen in der Alkylgruppe und einem mittleren Oligomerisationsgrad des Glycosidrestes von 1-3 in einer Menge von 0,025 bis 2,5 Gew.-% enthalten sind.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, dass sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Mund und Zahnpflege- und -reinigungsmittel können darüber hinaus mit besonderem Vorzug Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein.

Erfindungsgemäße Mund- und Zahnpflege- und -reinigungsmittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel, enthalten, sind erfindungsgemäß bevorzugt. Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose sowie Maltose und Dextrose.

Ein weiterer Gegenstand der vorliegenden Erfindung ist die Verwendung von erfindungsgemäßen Mitteln zur Reduzierung von Zahnbelag.

Ein weiterer Gegenstand der vorliegenden Erfindung ist ein Verfahren zum Reinigen von Zähnen, dadurch gekennzeichnet, dass ein erfindungsgemäßes Mittel auf den Bürstenkopf einer Zahnbürste aufgetragen und mit der Zahnbürste die Zähne geputzt werden.

Bezüglich bevorzugter Ausführungsformen der erfindungsgemäßen Verwendung und der erfindungsgemäßen Verfahren gilt mutatis mutandis das zu den erfindungsgemäßen Mitteln Gesagte.

### Beispiele:

Alle Angaben in Gew.-%.

### Beispiel 1: Zahnpasten

| | **1** | **2** | **3** | **4** | **5** | **6** |
|---|---|---|---|---|---|---|
| Sorbitol | 45 | 50 | 55 | 60 | 65 | 70 |
| Hydrated silica | 5 | 10 | 15 | 20 | 10 | 5 |
| Natriumflourid | 0,1 | 0,32 | 0,1 | 0,32 | 0,1 | 0,32 |
| Natrium Saccharin | 0,1 | 0,2 | 0,3 | 0,4 | 0,5 | 0,15 |
| H₂N-(CH₂)₃-NH-(CH₂)₃-NH₂ | 0,03 | 0,1 | 0,05 | 0,07 | 0,1 | 0,03 |
| Aroma | 0,1 | 0,25 | 0,5 | 1,5 | 0,2 | 0,15 |
| Ethanol | 5 | 0 | 0 | 0 | 0 | 0 |
| Xanthan gum | 0,2 | 0,3 | 0,4 | 0,5 | 0,2 | 0,1 |
| Natriumdodecylsulfat | 1 | 1,5 | 1,5 | 5,0 | 1,5 | 1,5 |
| Cocamidopropyl Betain | 0,5 | 0,6 | 1 | 0,6 | 0,5 | 0,5 |
| Wasser | ad 100 | ad 100 | ad 100 | ad 100 | ad 100 | ad 100 |

| | **7** | **8** | **9** | **10** | **11** | **12** |
|---|---|---|---|---|---|---|
| Sorbitol | 45 | 50 | 55 | 60 | 65 | 70 |
| Hydrated silica | 5 | 10 | 15 | 20 | 10 | 5 |
| Natriumflourid | 0,1 | 0,32 | 0,1 | 0,32 | 0,1 | 0,32 |
| Natrium Saccharin | 0,1 | 0,2 | 0,3 | 0,4 | 0,5 | 0,15 |
| H₂N-(CH₂)₃-NH-(CH₂)₄-NH₂ | 0,03 | 0,1 | 0,05 | 0,07 | 0,1 | 0,03 |
| Aroma | 0,1 | 0,25 | 0,5 | 1,5 | 0,2 | 0,15 |
| Ethanol | 5 | 0 | 0 | 0 | 0 | 0 |
| Xanthan gum | 0,2 | 0,3 | 0,4 | 0,5 | 0,2 | 0,1 |
| Natriumdodecylsulfat | 1 | 1,5 | 1,5 | 5,0 | 1,5 | 1,5 |
| Cocamidopropyl Betain | 0,5 | 0,6 | 1 | 0,6 | 0,5 | 0,5 |
| Wasser | ad 100 | ad 100 | ad 100 | ad 100 | ad 100 | ad 100 |

### Beispiel 2: Mouthwashes

| | **1** | **2** | **3** | **4** | **5** | **6** |
|---|---|---|---|---|---|---|
| Sorbitol | 1 | 2 | 3 | 4 | 5 | 1 |
| PEG-60 Hydrogenated Castor Oil | 0,1 | 0,5 | 1,0 | 0,2 | 0,4 | 0,1 |
| Natriumfluorid | 0,11 | 0,10 | 0,09 | 0,11 | 0,11 | 0,11 |
| Trinatriumcitrat Dihydrat | 0,1 | 0,2 | 0,3 | 0,4 | 0,5 | 0,6 |
| Citronensäure | 0,001 | 0,01 | 0 | 0 | 0,01 | 0,001 |
| Natrium Saccharin | 0,1 | 0,2 | 0,3 | 0,2 | 0,15 | 0,1 |
| Cetylpyridinium Chlorid | 0,01 | 0,1 | 0 | 0 | 0,01 | 0,05 |
| Aroma | 0,1 | 0,25 | 0,5 | 1,5 | 0,2 | 0,15 |
| Ethanaol | 0 | 10 | 30 | 0 | 5 | 5 |
| H₂N-(CH₂)₃-NH-(CH₂)₃-NH₂ | 0,03 | 0,1 | 0,05 | 0,07 | 0,1 | 0,03 |
| Wasser | ad 100 | ad 100 | ad 100 | ad 100 | ad 100 | ad 100 |

| | **7** | **8** | **9** | **10** | **11** | **12** |
|---|---|---|---|---|---|---|
| Sorbitol | 1 | 2 | 3 | 4 | 5 | 1 |
| PEG-60 Hydrogenated Castor Oil | 0,1 | 0,5 | 1,0 | 0,2 | 0,4 | 0,1 |
| Natriumfluorid | 0,11 | 0,10 | 0,09 | 0,11 | 0,11 | 0,11 |
| Trinatriumcitrat Dihydrat | 0,1 | 0,2 | 0,3 | 0,4 | 0,5 | 0,6 |
| Citronensäure | 0,001 | 0,01 | 0 | 0 | 0,01 | 0,001 |
| Natrium Saccharin | 0,1 | 0,2 | 0,3 | 0,2 | 0,15 | 0,1 |
| Cetylpyridinium Chlorid | 0,01 | 0,1 | 0 | 0 | 0,01 | 0,05 |
| Aroma | 0,1 | 0,25 | 0,5 | 1,5 | 0,2 | 0,15 |
| Ethanaol | 0 | 10 | 30 | 0 | 5 | 5 |
| H₂N-(CH₂)₃-NH-(CH₂)₄-NH₂ | 0,03 | 0,1 | 0,05 | 0,07 | 0,1 | 0,03 |
| Wasser | ad 100 | ad 100 | ad 100 | ad 100 | ad 100 | ad 100 |

Auf angezüchteten Biofilmen auf Glasoberflächen wiesen alle 24 Zusammensetzungen gegenüber Polyamin-freien Zusammensetzungen deutlich verbesserte Wirkungen auf.

## Patentansprüche

1. Mund- und Zahnpflege- und -reinigungsmittel, enthaltend - bezogen auf sein Gewicht - 0,00001 bis 0,1 Gew.-% mindestens eines Polyamins der Formel (I) 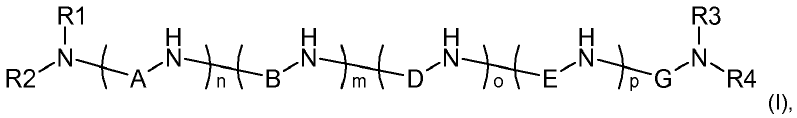 in der
R¹, R², R³ und R⁴ stehen unabhängig voneinander für -H, -CH₃, -CH₂CH₃, -CH₂CH₂CH₃, -CH(CH₃)₂;
n, m, o, p stehen unabhängig voneinander für 0, 1, 2, 3, 4, 5, wobei gilt: (n + m + o + p) > 0;
A, B, D, E, G stehen unabhängig voneinander für eine gesättigte oder ungesättigte Kohlenwasserstoffgruppierung.

2. Mund- und Zahnpflege- und -reinigungsmittel nach Anspruch 1, **dadurch gekennzeichnet, dass** es 0,0001 bis 0,09 Gew.-% vorzugsweise 0,001 bis 0,08 Gew.-% , weiter bevorzugt 0,01 bis 0,07 Gew.-%, noch weiter bevorzugt 0,02 bis 0,06 Gew.-% und insbesondere 0,025 bis 0,05 Gew.-% Polyamin(e) der Formel (I) enthält.

3. Mund- und Zahnpflege- und -reinigungsmittel nach einem der Ansprüche 1 oder 2, **dadurch gekennzeichnet, dass** es 0,0001 bis 0,09 Gew.-% vorzugsweise 0,001 bis 0,08 Gew.-% , weiter bevorzugt 0,01 bis 0,07 Gew.-%, noch weiter bevorzugt 0,02 bis 0,06 Gew.-% und insbesondere 0,025 bis 0,05 Gew.-% Polyamin(e) der Formel (I) enthält, in der n für 1 steht und gleichzeitig m = p gilt.

4. Mund- und Zahnpflege- und -reinigungsmittel nach einem der Ansprüche 1 bis 3, **dadurch gekennzeichnet, dass** es 0,0001 bis 0,09 Gew.-% vorzugsweise 0,001 bis 0,08 Gew.-% , weiter bevorzugt 0,01 bis 0,07 Gew.-%, noch weiter bevorzugt 0,02 bis 0,06 Gew.-% und insbesondere 0,025 bis 0,05 Gew.-% Polyamin(e) der Formel (I) enthält, in der A, B, D, E, G unabhängig voneinander definiert sind wie folgt:
-(CH₂)ₐ- mit a = 1, 2, 3, 4, 5, 6;
-(CH₂)_{b}-CH=CH-(CH₂)_{c}-, in der b und c unabhängig voneinander für 1, 2, 3, 4, 5, 6 stehen;
-(CH₂)_{d}-CH=CH-(CH₂)ₑ-CH=CH-(CH₂)_{f}-, in der d, e und f unabhängig voneinander für 1, 2, 3, 4, 5, 6 stehen.

5. Mund- und Zahnpflege- und -reinigungsmittel nach einem der Ansprüche 1 bis 4, **dadurch gekennzeichnet, dass** es 0,0001 bis 0,09 Gew.-% vorzugsweise 0,001 bis 0,08 Gew.-% , weiter bevorzugt 0,01 bis 0,07 Gew.-%, noch weiter bevorzugt 0,02 bis 0,06 Gew.-% und insbesondere 0,025 bis 0,05 Gew.-% Polyamin(e) der Formel (I) enthält, in der gilt:
- R¹ = R² = R³ = R⁴ = -H
- n = 1
- m = o = p = 0
- A und G sind unabhängig voneinander ausgewählt aus -(CH₂)₃-, -(CH₂)₄-, -(CH₂)-CH=CH-(CH₂)-, und -(CH₂)-CH=CH-(CH₂)-CH=CH-(CH₂)-.

6. Mund- und Zahnpflege- und -reinigungsmittel nach einem der Ansprüche 1 bis 5, **dadurch gekennzeichnet, dass** es 0,0001 bis 0,09 Gew.-% vorzugsweise 0,001 bis 0,08 Gew.-% , weiter bevorzugt 0,01 bis 0,07 Gew.-%, noch weiter bevorzugt 0,02 bis 0,06 Gew.-% und insbesondere 0,025 bis 0,05 Gew.-% Polymain(e) der Formeln 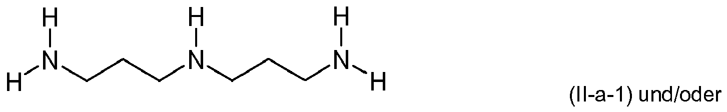 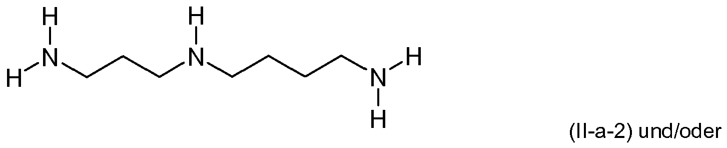 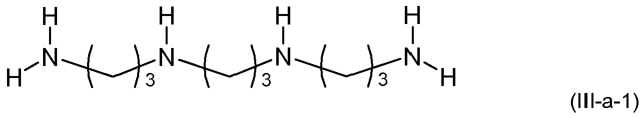 und/oder deren Mischungen enthält.

7. Mund- und Zahnpflege- und -reinigungsmittel nach einem der Ansprüche 1 bis 5, **dadurch gekennzeichnet, dass** es 0,0001 bis 0,09 Gew.-% vorzugsweise 0,001 bis 0,08 Gew.-% , weiter bevorzugt 0,01 bis 0,07 Gew.-%, noch weiter bevorzugt 0,02 bis 0,06 Gew.-% und insbesondere 0,025 bis 0,05 Gew.-% Polymain(e) der Formel (IVa-1) bis (IVa-10) und/oder deren Mischungen enthält: 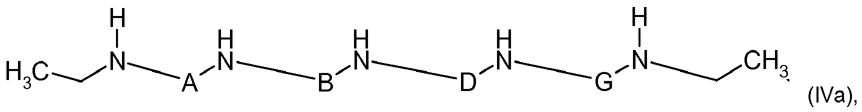 in der gilt:
A = B = D = G = -(CH₂)₄- (IVa-1) und/oder
A = B = D = -(CH₂)₄-, G = -(CH₂)-CH=CH-(CH₂)- (IVa-2) und/oder
A = B = G = -(CH₂)₄-, D = -(CH₂)-CH=CH-(CH₂)- (IVa-3) und/oder
A = B = -(CH₂)₄-, D = G = -(CH₂)-CH=CH-(CH₂)- (IVa-4) und/oder
A = G = -(CH₂)₄-, D = B = -(CH₂)-CH=CH-(CH₂)- (IVa-5) und/oder
D = B = -(CH₂)₄-, A = G = -(CH₂)-CH=CH-(CH₂)- (IVa-6) und/oder
A = D = -(CH₂)₄-, B = G = -(CH₂)-CH=CH-(CH₂)- (IVa-7) und/oder
A = -(CH₂)₄-, B = D = G = -(CH₂)-CH=CH-(CH₂)- (IVa-8) und/oder
B = -(CH₂)₄-, A = D = G = -(CH₂)-CH=CH-(CH₂)- (IVa-9) und/oder
A = B = D = G = -(CH₂)-CH=CH-(CH₂)- (IVa-10).

8. Mund- und Zahnpflege- und -reinigungsmittel nach einem der Ansprüche 1 bis 7, **dadurch gekennzeichnet, dass** es zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper,vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthält.

9. Verwendung von Mitteln nach einem der Ansprüche 1 bis 8 zur Reduzierung von Zahnbelag.

10. Verfahren zum Reinigen von Zähnen, **dadurch gekennzeichnet, dass** ein Mittel nach einem der Ansprüche 1 bis 8 auf den Bürstenkopf einer Zahnbürste aufgetragen und mit der Zahnbürste die Zähne geputzt werden.
